# EUROPEAN PATENT SPECIFICATION

(11) **EP 0 353 361 B1**
(45) Date of publication and mention of the grant of the patent: **28.04.1993**
(21) Application number: 88307227.4
(22) Date of filing: 05.08.1988
(51) Int. Cl.: G01N 33/68, G01N 33/52

(54) **A method for detecting amino acid derivatives**
Verfahren zum Nachweis von Aminosäure-Derivaten
Procédé pour la détection des dérivés des aminoacides

(43) Date of publication of application: 07.02.1990
(73) Proprietor: SEIKO INSTRUMENTS INC., Tokyo 136 (JP)
(72) Inventor: Tsugita, Akira, Kashiwa-shi Chiba-ken (JP); Uchida, Toyoaki, Koto-ku Tokyo (JP)
(74) Representative: Petersen, Richard Courtenay

(56) References cited:
- EP-A- 0 202 894
- AGR. BIOL. CHEM., vol. 40, no. 4, 1976, pages 815-817; K. MURAMOTO et al.: "Analysis of fluorescein-thiohydantoin amino acids by high speed liquid chromatography"
- ANALYTICAL BIOCHEMISTRY, vol. 77, 1977, pages 569-573, Academic Press; C.L. ZIMMERMAN et al.: "Rapid analysis of amino acid phenylthiohydantoins by high-performance liquid chromatography"

## Description

This invention relates to methods for detecting amino acid derivatives, for example, for application to sequence determination from amino (N) terminal of a protein.

It is known to detect amino acids using the phenylisothiocyanate method according to Edman, P. Acta. Chem. Scand. 10 761 (1956). The Edman method is an N terminal sequence determination method. In the final step of the Edman method, it has been usual practice to treat thiazolinone derivatives with an acid to form phenylthiohydantoin (PTH) derivatives and to determine these derivatives spectrophotometrically. Although this known method for spectrophotometrically detecting PTH derivatives is simple and convenient, it cannot fully cope with a recent trend towards more highly sensitive analysis of a protein with a smaller amount of specimen.

For high sensitivity detection of amino acids, methods using ³²S phenylisothiocyanate labelling or ¹³⁵I phenylisothiocyanate labelling are disclosed in - W. G. Lauer, Fundamental Techniques in Virology, Eds. K. Habel and N. P. Salgman. Pages 379 (1961) Academic Press. N.Y.,
C. J. Burrell, P. D. Cooper, J. M. Swann, Aus. J. Chem. 28 2289 (1975).

In the disclosed methods, radio-active isotope derivatives take part in the main reaction in the Edman degradation method. However, if high radio-activity is used in order to obtain high sensitivity, radio-active disintegration increases which adversely affects yield in the Edman amino acid sequence determination, and also the contamination of environment occurs.

Fluorescein may be attached to the isothiocyanate group of phenyl isothiocyanate for use in an Edman degradation as disclosed in K. Muramoto et al., Agr. Biol. Chem., 40(4), 815 (1976).

EP 202 894 relates to a method for detecting amino acid derivatives by reacting 5-thiazolinone amino acid derivatives with an amino compound labelled wth a radioactive iodine or with a fluorescent compound such as fluorene or pyrene.

The present invention seeks to provide a method for detecting amino acid derivatives which, when applied to amino acid sequence determination from the amino terminal of a protein, results in higher sensitivity and no contamination of the environment.

According to one aspect of the present invention, there is provided a method for detecting an amino acid derivative which method is characterised by comprising the steps of: reacting a 2-anilino-5-thiazolinone derivative of the general formula (a) below with an amino compound of fluorescein or an amino compound of rhodamine derivatives of the general formula (b) below to form a phenylthiocarbamyl amino acid derivative of the general formula (c) below; and detecting said phenylthio-carbamyl amino acid derivative 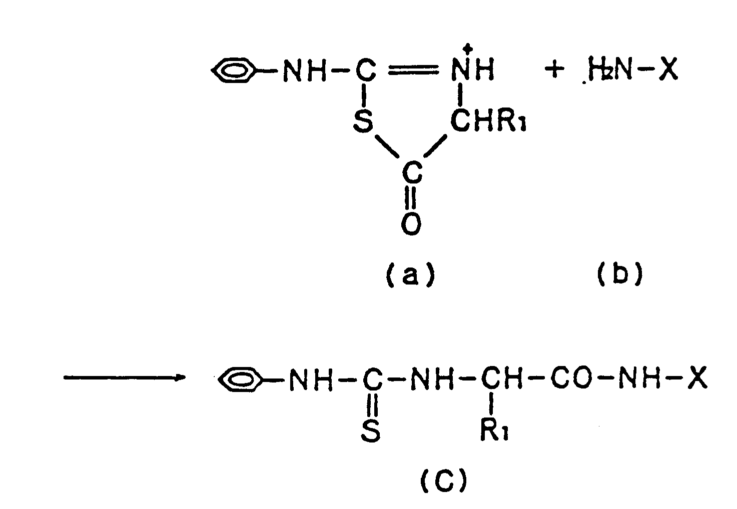
wherein X is a fluorescein or a rhodamine derivative and R₁ is an amino acid side chain.

According to another aspect of the present invention there is provided a method for detecting an amino acid derivative which method is characterised by comprising the steps of: reacting phenylisothiocyanate of the general formula (d) below with a protein or a peptide of the general formula (e) below to form a phenylthiocarbamylprotein or a phenylthiocarbamylpeptide of the general formula (f) below; reacting said phenylthiocarbamylprotein or phenylthiocarbamylpeptide with an acid in an anhydrous condition to effect cyclization and scission to form a 2-anilino-5-thiazolinone derivative of the general formula (a) below; reacting said 2-anilino-5-thiazolinone derivative with an amino compound of fluorescein or an amino compound of rhodamine derivatives of the general formula (b) below to form a phenylthiocarbamyl amino acid derivative of the general formula (c) below; and detecting said phenylthiocarbamyl amino acid derivative 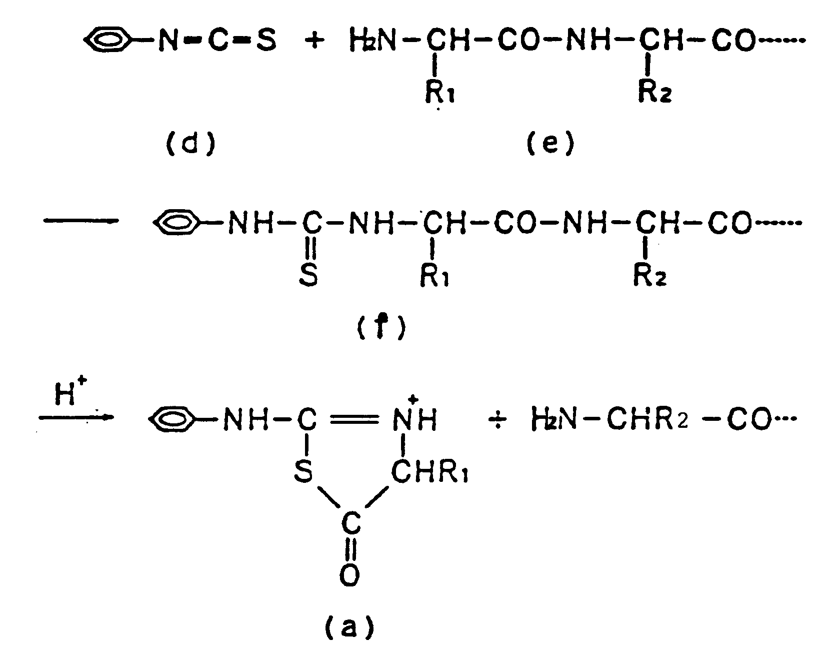 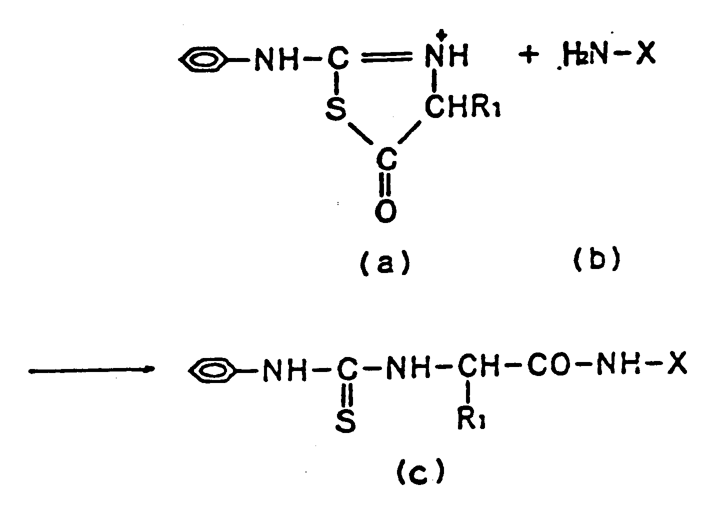
wherein X is an fluorescein or rhodamine derivative and R₁ and R₂ are amino acid side chains.

The phenylthiocarbamyl amino acid derivative may be detected using a fluorescence spectrophotometer.

The invention is illustrated, merely by way of example, in the accompanying drawings, in which:-
Figure 1 is a process flow diagram showing a detection method according to the present invention;
Figure 2 is a process flow diagram of a known detection method;
Figure 3 shows two high pressure liquid chromatography (HPLC) charts or graphs of a reaction mixture and a reaction product, the charts being such that:
Figure 3 (i) illustrates unreacted thiazolinone, and
Figure 3 (ii) illustrates the reaction product after mixing at room temperature for three minutes;
Figure 4 is an HPLC chart of a phenylthiocarbamyl derivative of leucine; and
Figure 5 is an HPLC chart of a mixture of various phenylthiocarbamyl amino acid derivatives.

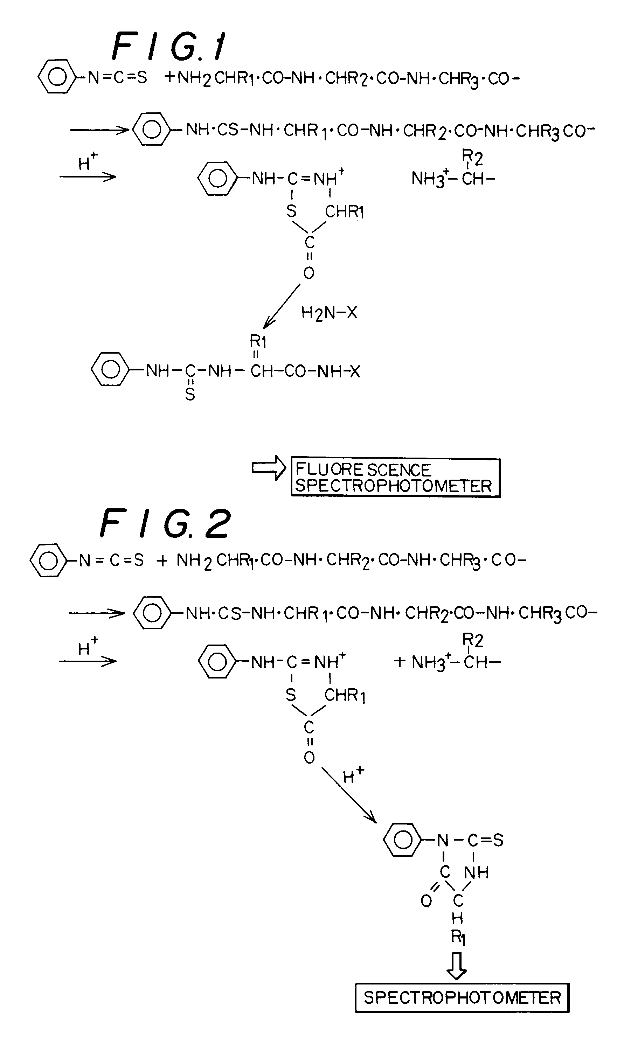

Figure 1 shows a process flow diagram of a detection method according to the present invention. The method according to the present invention comprises the steps of reacting a 2-anilino-5-thiazolinone derivative with a fluorescent amino compound such as aminofluorescein or rhodamine derivative amino compound, to form a phenylthiocarbamyl (PTC) amino acid derivative and detecting the amino acid derivative with high sensitivity by making use of a fluorophotometer or a fluorescence spectrophotometer. In Figure 1, each of R₁, R₂ and R₃ is an amino acid side chain and X denotes fluorescein or rhodamine derivative.

In order to facilitate a full and complete understanding of the present invention, reference will also be made to the following Examples.

### EXAMPLE 1

This Example illustrates a basic detection method according to the present invention wherein a 2-anilino-5-thiazolinone derivative of an amino acid (ATZ) is reacted with 4-aminofluorescein to form a phenylthiocarbamyl (PTC) amino acid derivative and the reaction product is detected with very high sensitivity.

In this Example, a dipeptide hereinafter abbreviated as (Ala-Gly) consisting of glycine (hereinafter abbreviated as Gly) and alanine (hereinafter abbreviated as Ala) is used instead of a protein for simplicity. The basic detection method is described in the following order:

### i) Synthesis and formulation of ATZ-Ala (see Molecular Biology, Biochemistry and Biophysics, volume 8, "Protein Sequence Determination" edited by Saul B. Neadleman, Springer-Verlag, page 253).

The reaction proceeds according to the following reaction schemes [IV] and [V]:

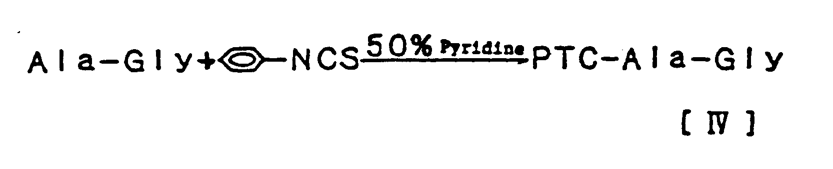

Ala-Gly was dissolved in a 50% aqueous pyridine solution, and 2M NaOH was added thereto to adjust the pH value to 8.6. Subsequently, phenylisothiocyanate (PITC) was added thereto. Since the pH value lowered with the addition of the PITC, 2M NaOH was added to maintain the pH value at 8.6. After the variation in the pH value had been decreased, the solution was heated at 40°C for one hour. After completion of the reaction, the reaction mixture was washed with benzene. After the benzene dissolved in the water phase had been purged with nitrogen (N₂) gas, the pH was adjusted to 2 by the addition of 1M HCl, thereby preparing PTC-Ala-Gly in the form of a white precipitate.

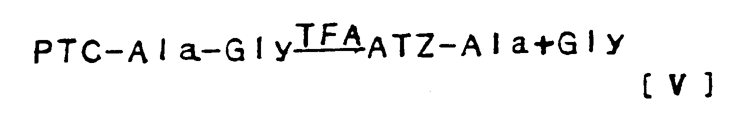

PTC-Ala-Gly thus prepared was dissolved in TFA, and the solution was heated at 50°C for five minutes. After the completion of the reaction, the reaction mixture was evaporated to dryness. Butyl chloride was added to the residue to thoroughly dissolve the product therein, and the resulting solution was passed through a cellulose column (Gly was adsorbed on the column). The effluent was collected and evaporated to dryness, thereby preparing ATZ-Ala in the form of a white crystalline solid.

### ii) Coupling reaction

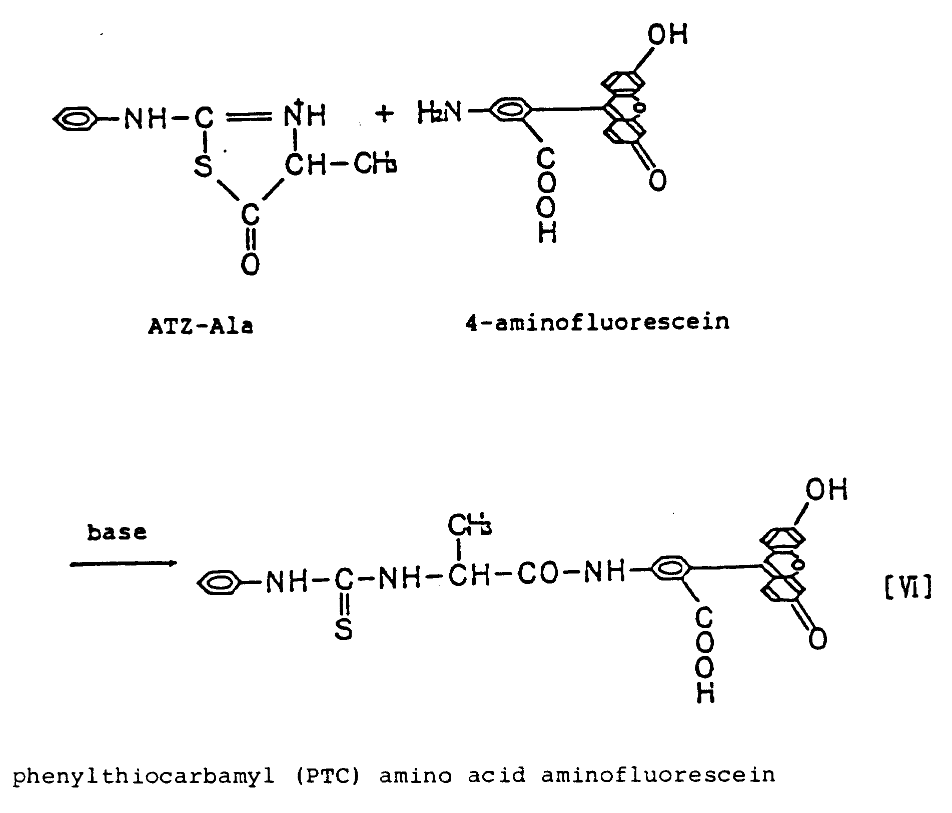

As shown in the above reaction scheme [VI], the amino group of 4-aminofluorescein attacks the carbonyl group of the ATZ amino acid. This reaction is the reverse of a reaction for cleaving a peptide bond between PITC and the peptide. The reaction was conducted at room temperature for three minutes in a solvent comprising pyridine (10%) and dimethylformamide.

### iii) Detection of phenylthiocarbamyl amino acid

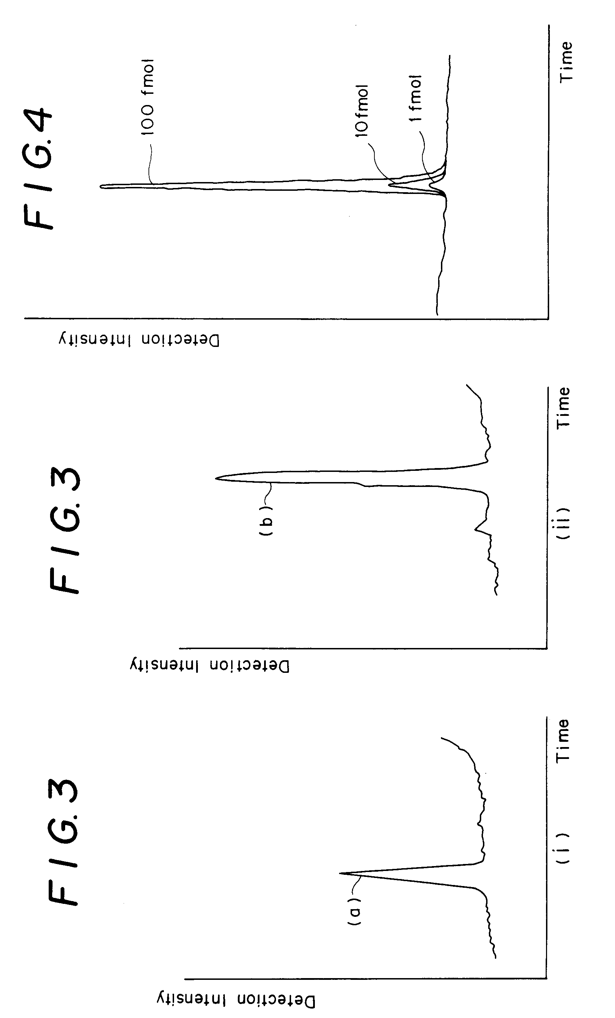

The reaction mixture was applied to a high pressure liquid chromatography (HPLC) column, and detection was made with ultra-violet absorption spectra (at 269 nm). The results are shown in Figure 3.

Figure 3 (i) is an HPLC chromatogram of a thiazolinone derivative, while Figure 3 (ii) is an HPLC chromatogram after the reaction of the thiazolinone derivative for three minutes. In Figure 3 (i) and 3 (ii), (a) is a peak of a thiazolinone derivative of alanine (Ala) and (b) is a peak of the phenylthiocarbamyl derivative of alanine. As can be seen, the coupling reaction between the thiazolinone derivative of alanine and 4-aminofluorescein was completed in three minutes at room temperature, and the thiazolinone derivative was detected in the form of a thiocarbamylalanine derivative.

### (Conditions of HPLC)

column:
SERVA Cat. No. 42318
250 mm x 4.6 mm
SERVACHROM® Packing
solvent system:

| buffer solution | organic solvent |
|---|---|
| 0.015 M | CH₃CN-MeOH |
| NaOAc + AcOH | (4 : 1) |
| pH 5 | |
| 1 | 5 |

effluent flow rate: 1.5 ml/min
detection wavelength: 269 nm

### EXAMPLE 2

This Example show the results of an experiment wherein a 2-anilino-5-thiazolinone derivative of an amino acid was reacted with 4-aminofluorescein to form a phenylthiocarbamyl amino acid derivative. The maximum sensitivity with which the amino acid derivative can be detected was investigated. The phenylthiocarbamyl leucine (Leu) derivative prepared in the same way as in Example 1 from a dipeptide (Leu-Gly) was dissolved in methanol, and 1 femto (10⁻¹⁵) mol, 10 femto mol and 100 femto mol (10 µl) thereof were applied to an HPLC column and detected with a fluorophotometer (an excitation wavelength of 500 nm and a fluorescence wavelength of 516 nm) respectively. The results are shown in Figure 4.

The comparison of sensitivity of detection according to the present invention with that of the conventional phenylthiohydantoin amino acid detection method (PTH method) gave the following results, i.e., revealed that the sensitivity of detection according to the present invention was much higher than that according to the known method (Figure 2).

| detection method | sensitivity of detection |
|---|---|
| PTH method | less than 10 pico mol |
| present invention | 1 femto mol |

### (Condition of HPLC)

- column:: SHISEIDO CAPSEL® PAC C18 AG Type
150 mm x 4.6 mm
- solvent system:: A: 10 mM Na₂HPO₄ - NaH₂PO₄; pH 8.0
B: MeOH
gradient elution
flow rate: 1 ml/min
temperature: 40 °C
- detection:: excitation wavelength: 486 nm
fluorescence wavelength: 513 nm

### EXAMPLE 3

This Example illustrates how a mixture of phenylthiocarbamyl amino acid derivatives of amino acids constituting a protein was separated into components and detected by HPLC (the conditions of HPLC were the same as those of Example 2).

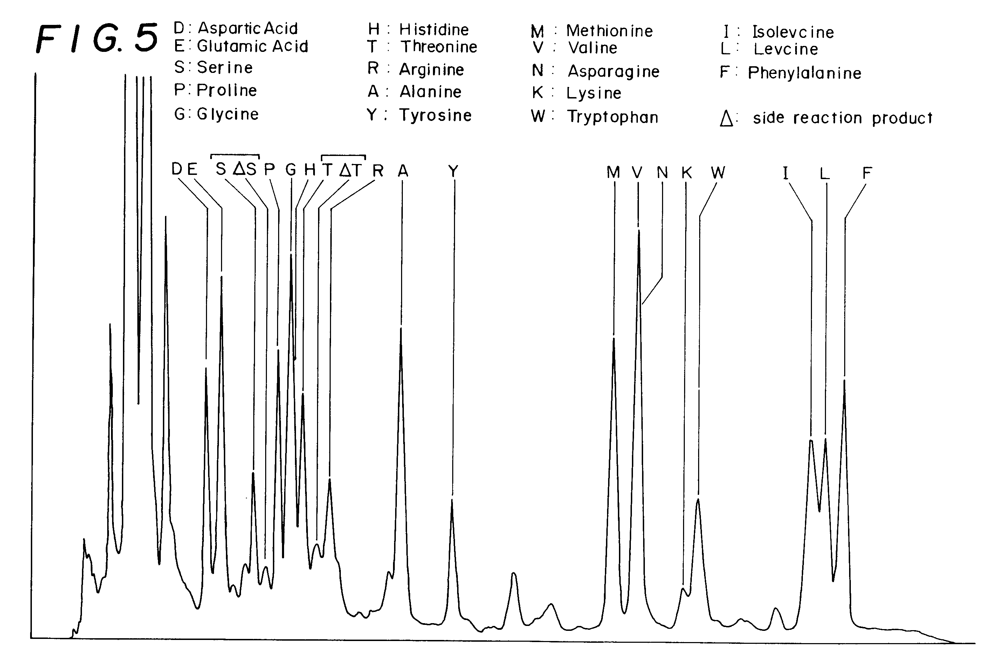

A mixture of phenylthiocarbamyl amino acid derivatives synthesized by making use of dipeptides containing predetermined amino acids according to the procedures described in Example 1 was analysed by HPLC under the conditions shown in Example 2. The results are shown in Figure 5. As can be seen, the phenylthiocarbamyl derivatives of 20 amino acids which are components constituting a protein were excellently separated.

It will be appreciated from the above Figures and Examples that the present invention resides in reacting a fluorescent amino compound with thiazolinone derivative of an amino acid to form a phenylthiocarbamyl derivative of the amino compound and the amino acid derivative is detected without using any radio-isotope.

In the above Examples, 4-aminofluorescein was used, but it will be understood that other fluorescent amino compounds can be used, and that the present invention is not limited to the particular chemicals or compounds used in the Examples. As will be apparent from the foregoing description, the method according to the present invention for detecting an amino acid derivative with high sensitivity is very useful from the point of view of industry.

## Claims

1. A method for detecting an amino acid derivative which method is characterised by comprising the steps of: reacting a 2-anilino-5-thiazolinone derivative of the general formula (a) below with an amino compound of fluorescein or an amino compound of rhodamine derivatives of the general formula (b) below to form a phenylthiocarbamyl amino acid derivative of the general formula (c) below; and detecting said phenylthiocarbamyl amino acid derivative 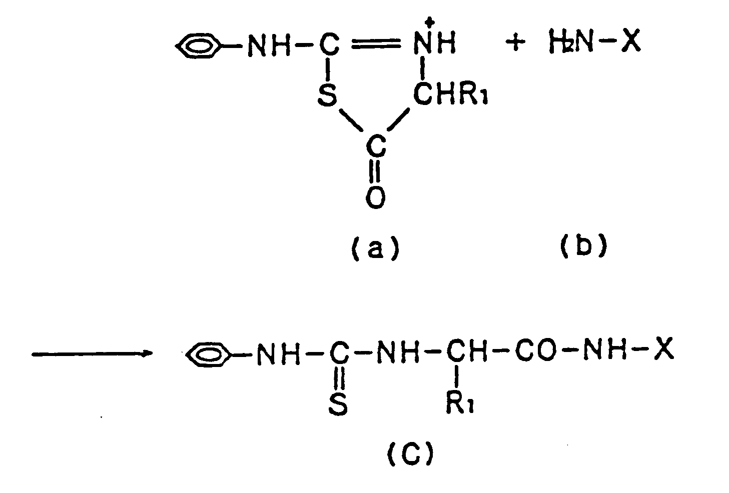 wherein X is a fluorescein or a rhodamine derivative and R₁ is an amino acid side chain.

2. A method for detecting an amino acid derivative which method is characterised by comprising the steps of: reacting phenylisothiocyanate of the general formula (d) below with a protein or a peptide of the general formula (e) below to form a phenylthiocarbamylprotein or a phenylthiocarbamylpeptide of the general formula (f) below; reacting said phenylthiocarbamylprotein or phenylthiocarbamylpeptide with an acid in an anhydrous condition to effect cyclization and scission to form a 2-anilino-5-thiazolinone derivative of the general formula (a) below; reacting said 2-anilino-5-thiazolinone derivative with an amino compound of fluorescein or an amino compound of rhodamine derivatives of the general formula (b) below to form a phenylthiocarbamyl amino acid derivative of the general formula (c) below; and detecting said phenylthiocarbamyl amino acid derivative 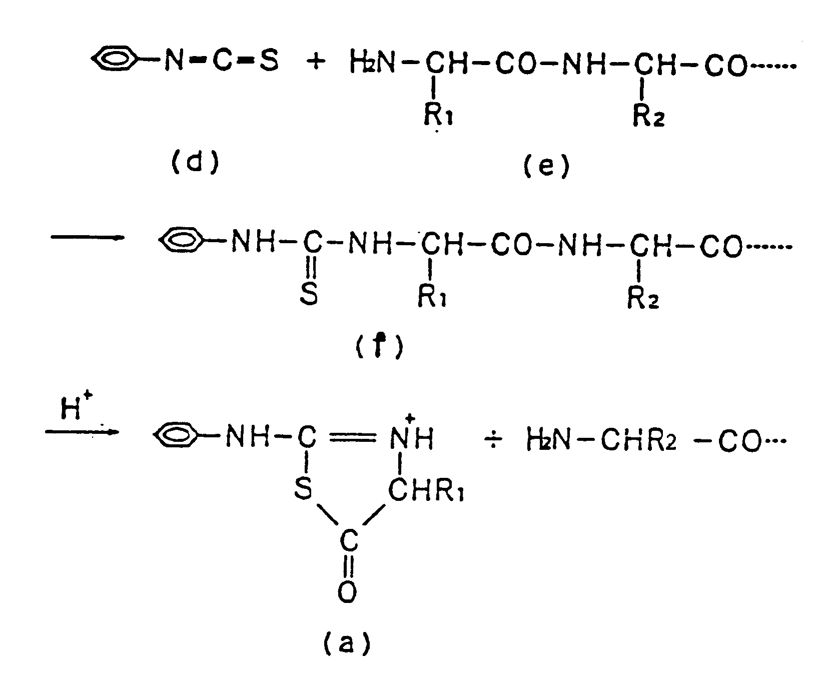 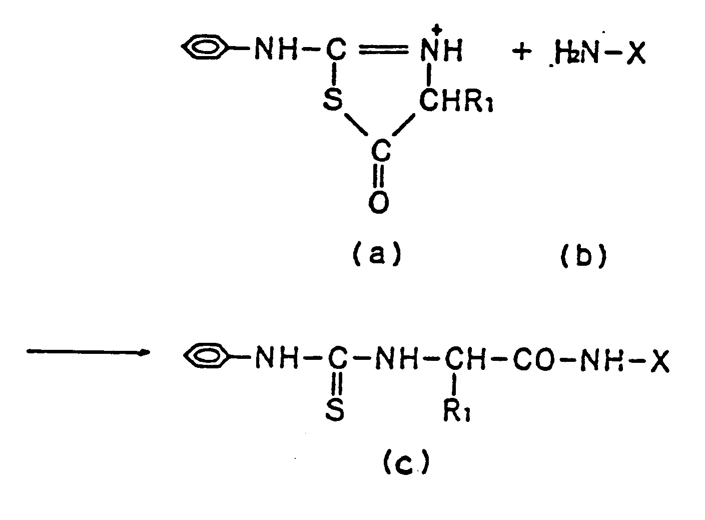 wherein X is an fluorescein or rhodamine derivative and R₁ and R₂ are amino acid side chains.

3. A method as claimed in claim 1 or 2 in which the phenylthiocarbamyl amino acid derivative is detected using a fluorescence spectrophotometer.

## Patentansprüche

1. Verfahren zur Bestimmung eines Aminosäurenderivates, dadurch gekennzeichnet, daß zur Bildung eines Phenylthiocarbamylaminosäuren-Derivates der unten angegebenen generellen Formel (c) ein 2-Anilino-5-Thiazolinon-Derivat der unten angegebenen generellen Formel (a) mit einer Aminoverbindung von Floureszeinderivaten oder einer Aminoverbindung von Rhodaminderivaten der unten angegebenen generellen Formel (b) zur Reaktion gebracht wird und daß das Phenylthiocarbamylaminosäuren-Derivat bestimmt wird, 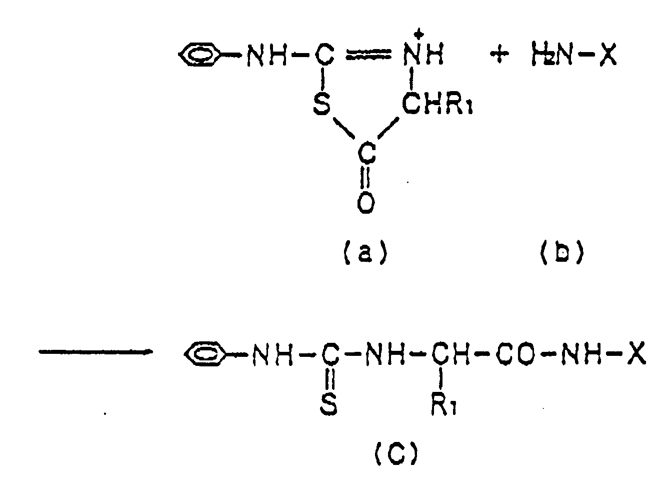 worin X ein Fluoreszein- oder ein Rhodaminderivat und R₁ eine Aminosäuren-Seitenkette bedeuten.

2. Verfahren zur Bestimmung eines Aminosäurenderivates, dadurch gekennzeichnet, daß zur Bildung eines Phenylthiocarbamylproteins oder eines Phenylthiocarbamylpeptides der unten angegebenen generellen Formel (f) ein Phenylisothiocyanat der unten angegebenen generellen Formel (d) mit einem Protein oder einem Peptide der unten angegebenen generellen Formel (e) zur Reaktion gebracht wird, daß zur Realisierung einer Ringbildung und Spaltung für die Bildung eines 2-Anilino-5-Thiazolinon-Derivates der unten angegebenen generellen Formel (a) das Phenylthiocarbamylprotein oder Phenyltriocarbamylpeptid in einem wasserfreien Zustand mit einer Säure zur Reaktion gebracht wird, daß zur Bildung eines Phenylthiocarbamylaminosäurenderivates der unten angegebenen generellen Formel (c) das 2-Anilino-5-Thiazolinon-Derivat mit einer Aminoverbindung von Fluoreszein-oder einer Aminoverbindung von Rhodaminderivaten der unten angegebenen generollen Formel (b) zur Reaktion gebracht wird und daß das Phenylthiocarbamylaminosäuren-Derivat bestimmt wird, 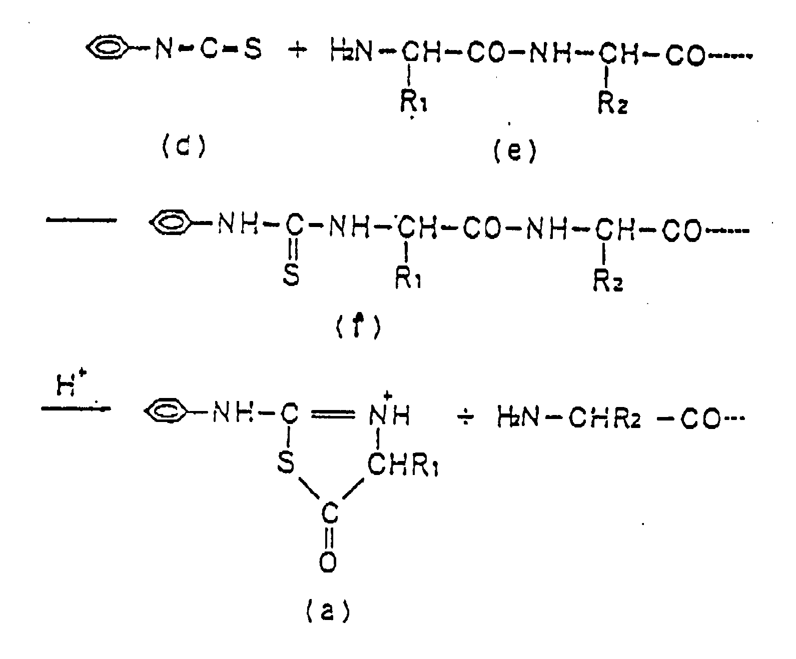 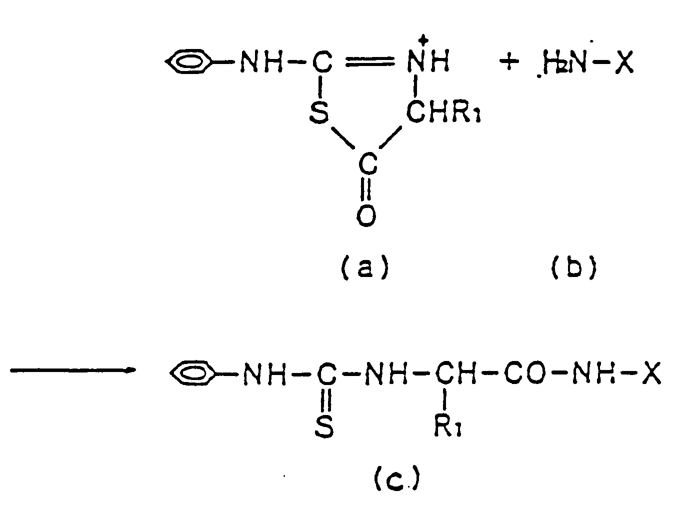 worin X ein Fluoreszin- oder Rhodaminderivat und R₁ und R₂ Aminosäuren-Seitenketten bedeuten.

3. Verfahren nach Anspruch 1 oder 2, bei dem das Phenylthiocarbamylaminosäuren-Derivat unter Verwendung eines Fluoreszensspektrophotometers bestimmt wird.

## Revendications

1. Procédé de détection d'un acide aminé, caractérisé en ce qu'il consiste à faire réagir une 2-anilino-5-thiazolinone de formule développée (a) ci-dessous sur un composé aminé de fluorescéine ou sur un composé aminé de rhodamine de formule développée (b) ci-dessous pour former un acide phénylthiocarbamyle aminé de formule développée (c) ci-dessous; et à détecter cet acide phénylthiocarbamyle aminé 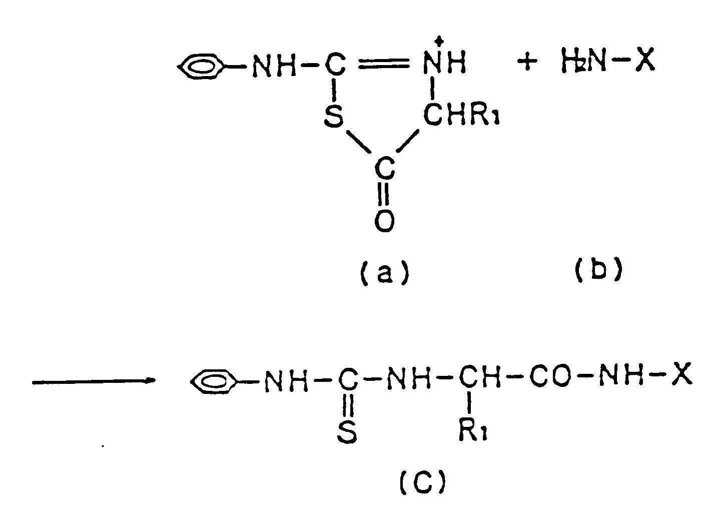 dans lesquelles X est la fluorescéine ou un dérivé de rhodamine et R₁ est une chaîne latérale acide aminée.

2. Procédé de détection d'un acide aminé, caractérisé en ce qu'il consiste à faire réagir un phénylisothiocyanate de formule développée (d) ci-dessous sur une protéine ou sur un pectine de formule développée (e) ci-dessous pour former une phénylthiocarbamylprotéine ou un phénylthiocarbamylpeptide de formule développée (f) ci-dessous; à faire réagir cette phénylthiocarbamylprotéine ou ce phénylthiocarbamylpeptide sur un acide dans des conditions anhydres pour effectuer une cyclisation et une scission et obtenir une 2-anilino-5-thiazolinone de formule développée (a) ci-dessous; à faire réagir cette 2-anilino-5-thiazolinone sur un composé aminé de fluorescéine ou sur un composé aminé de dérivés de rhodamine de formule générale (b) ci-dessous pour obtenir un acide phénylthiocarbamyle aminé de formule développée (c) ci-dessous; et à détecter cet acide phénylthiocarbamyle aminé 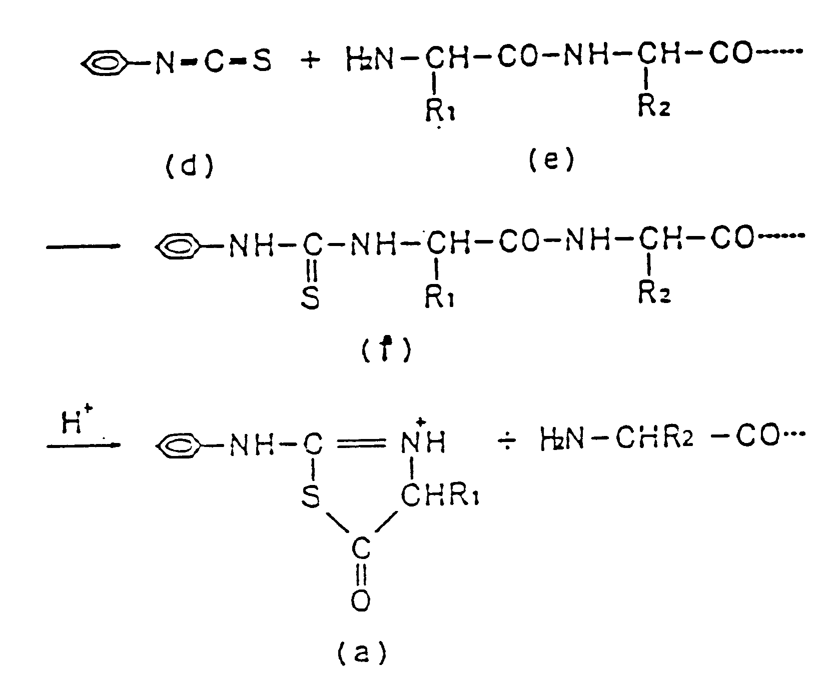 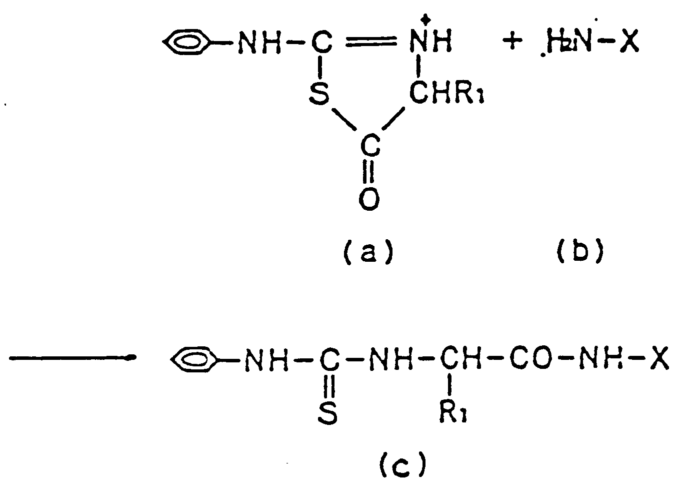 dans lesquelles X est de la fluorescéine ou un dérivé de rhodamine et R₁ et R₂ sont des chaînes latérales d'acide aminé.

3. Procédé suivant la revendication 1 ou 2, qui consiste à détecter l'acide phénylthiocarbamyle aminé en utilisant un spectrophotomètre à fluorescence.
